(19) Europäisches Patentamt
European Patent Office
Office européen des brevets

(11) **EP 2 106 852 B1**

(12) **EUROPEAN PATENT SPECIFICATION**

(45) Date of publication and mention
of the grant of the patent:
**31.10.2012 Bulletin 2012/44**

(51) Int Cl.:
*B01J 23/88* (2006.01)    *B01J 37/04* (2006.01)
*B01J 37/08* (2006.01)    *C07C 5/333* (2006.01)
*C07C 15/46* (2006.01)    *C07B 61/00* (2006.01)

(21) Application number: **08703896.4**

(22) Date of filing: **25.01.2008**

(86) International application number:
**PCT/JP2008/051069**

(87) International publication number:
**WO 2008/090974 (31.07.2008 Gazette 2008/31)**

(54) **CATALYST FOR DEHYDROGENATION OF ALKYL AROMATIC COMPOUND WHICH HAS IMPROVED PHYSICAL PROPERTIES, METHOD FOR PRODUCTION OF THE CATALYST, AND DEHYDROGENATION METHOD**

KATALYSATOR ZUR DEHYDRIERUNG EINER AROMATISCHEN ALKYLVERBINDUNG MIT VERBESSERTEN PHYSIKALISCHEN EIGENSCHAFTEN, VERFAHREN ZUR HERSTELLUNG DES KATALYSATORS UND DEHYDRIERUNGSVERFAHREN

CATALYSEUR POUR LA DÉSHYDROGÉNATION D'UN COMPOSÉ ALKYL AROMATIQUE QUI A DES PROPRIÉTÉS PHYSIQUES AMÉLIORÉES, PROCÉDÉ DE FABRICATION DU CATALYSEUR ET PROCÉDÉ DE DÉSHYDROGÉNATION

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.01.2007   JP 2007017203**

(43) Date of publication of application:
**07.10.2009 Bulletin 2009/41**

(73) Proprietor: **Sued-Chemie Catalysts Japan, Inc.**
**Shibuya-ku**
**Tokyo 151-0053 (JP)**

(72) Inventor: **MISHIMA, Yuji**
**Toyama-shi**
**Toyama 939-2753 (JP)**

(74) Representative: **Stolmár, Matthias et al**
**Stolmár Scheele & Partner**
**Patentanwälte**
**Blumenstrasse 17**
**80331 München (DE)**

(56) References cited:
**EP-A1- 0 297 657     JP-A- 10 502 617**
**JP-A- 58 177 148     JP-A- 2000 167 396**

**Description**

Technical Field of the Invention

This invention relates to a dehydrogenation catalyst having improved physical strength, process for producing the catalyst and dehydrogenation method thereof, in a catalyst used in a production of vinyl aromatic compounds, mainly styrene monomer, by dehydrogenating alkyl aromatic compounds, mainly ethylbenzene, in the presence of water vapor.

Technical Background

A styrene monomer is normally produced by dehydrogenating ethylbenzene, and it is utilized as a material monomer for synthetic rubber, ABS (acrylonitrile-butadiene-styrene) resin, polystyrene and the like, therefore, the production volume is increasing yearly.

A dehydrogenation reaction of ethylbenzene is an endothermic reaction accompanied by a volume expansion as shown in the formula below.

$$C_6H_5 - C_2H_5 \rightarrow C_6H_5 - C_2H_3 + H_2 + 30 \text{ kcal/mol}$$

The dehydrogenation reaction was studied intensively in the 1940s in the U.S.A. to meet the social demands for the production of synthetic rubber. During the period, a system of dehydrogenating ethylbenzene with contact under steam dilution was established technically, which is now employed globally and it has become a typical method for production of styrene.

Because of the volume expansion in the reaction, steam diluting of a reactant gives an advantage in chemical equilibration. Moreover, steam dilution has the following advantages.

(a) As the reaction is carried out at the high temperature of 550 - 650 °C, steam can be utilized as a heat source for heating ethylbenzene.

(b) Water gas reaction with steam can be utilized to remove the carbonaceous matters which are separated out by side reaction, by which a catalyst is revived and the catalyst can be used continuously.

(c) Steam as a dilution agent can be separated from a product easily by merely liquefying the product.

As described above, the dehydrogenation reaction system in the presence of steam is an industrially excellent production method for continuous production of styrene under the advantageous condition in chemical equilibration. This kind of operation method has become possible technically by confirming that a dehydrogenation catalyst comprising iron oxide and potassium oxide used in the reaction maintains a stable high performance. Further more attempts had been made to improve the performance of the catalyst until the catalyst became industrially utilizable, in particular, a number of catalyst compositions and adding promoters had been studied. For example, it has been found that high cerium-containing iron oxide and potassium oxide dehydrogenation catalysts containing 11 - 50 % by weight of $Ce_2O_3$ show high ethylbenzene conversion rate and styrene yield, and that increasing the amount of cerium compounds as basic compounds is an effective measure to improve the performance (see Patent Document 1).

Also, in producing styrene using dehydrogenation catalyst in industrial scale, fixed-bed reactors are used in most cases, and extruded moldings of pellets in various forms such as cylindrical form of 2.5 - 6 mm in diameter or gear form and the like are often used. Because of that, when the catalytic pellets do not have sufficient physical strength, pulverization and deterioration occur in the catalytic pellets while loading the catalyst into a reactor or during the operation of a reactor, catalytic activity and selectivity are decreased by increasing pressure loss of the reactor, and then it results not only in decrease of styrene yield but also in discontinuing production because of the abeyance of the reactor, therefore, improvement of physical strength of catalytic pellets is one of the most desired requirement in industrial catalyst as well as the performance improvement.

Furthermore, in case that a dehydrogenation catalyst is produced in industrial scale, carbonates, oxides, hydroxides and the like are used as a source of cerium because of the time, labor in handling and the cost reduction. In case that a high cerium-containing dehydrogenation catalyst comprising 5 - 35 % by weight of $CeO_2$ is prepared, the performance is improved as expected, however, the physical strength becomes too low to use in industrial scale.

Therefore, it is disclosed by Sherrod et al. that 3 - 20 % by weight of cement binding agents such as Portland cement or aluminate cement and the like are used in a dehydrogenation catalyst comprising iron oxide and potassium oxide containing 10 - 60 % by weight of $Ce_2O_3$ in order to improve the physical strength of catalytic pellets (see Patent Document 2).

Also, it is disclosed by Dellinger et al. that 0.2 -10 % of sodium compounds as sodium oxide and 1.5 - 20 % of calcium compounds as calcium oxide are used in addition to the cement binding agents in order to improve the physical strength of a high cerium-containing dehydrogenation catalyst comprising iron oxide and potassium oxide (see Patent Document 3).

EP 0297657A1 is directed to a dehydrogenation catalyst. For forming the dehydrogenation catalyst, an intimate mixture was prepared starting from red iron oxide, potassium carbonate, cerium carbonate and other components.

However, there still exists a problem that the catalyst activity will deteriorate considerably when cement binding agents or sodium compounds are added, compared to the products wherein those are not added.

Accordingly, it has been very difficult to produce a dehydrogenation catalyst containing high cerium having sufficient physical strength and quality by a conventional process.

Meanwhile, cerium carbonate hydroxide is known as a material of cerium oxide which has excellent absorption and releasing capacities of oxygen (see Patent Document 4). Also, it is well known that cerium carbonate hydroxide is widely used as a material for producing cerium (III) compounds (see Patent Document 5). Furthermore, the use is very limited. It is only used in a cerium abrading agent and also as a material of high functional cerium compounds (see Patent Document 6). In other words, in the conventional process, cerium carbonate hydroxide has rarely been used actively as a source of cerium for a dehydrogenation catalyst, moreover, it has never been used for the purpose of improving the physical strength of a dehydrogenation catalyst.

Patent Document 1: Japanese patent publication No. 3-11812
Patent Document 2: United States patent No. 4,758,543, Specification
Patent Document 3: United States patent No. 5,376,613, Specification
Patent Document 4: Japanese laid-open patent publication No. 5-105428
Patent Document 5: Japanese laid-open patent publication No. 2000-159521
Patent Document 6: Japanese laid-pen patent publication No. 2003-238948

DISCLOSURE OF INVENTION

PROBLEMS TO BE SOLVED BY THE INVENTION

The object of the present invention is to solve the above-mentioned conventional technical problem and to provide a dehydrogenation catalyst for dehydrogenating alkyl aromatic compounds, comprising iron oxide and potassium oxide containing high cerium, having improved physical strength of catalyst pellets used in industrial scale and a method for producing the catalyst, and the dehydrogenation method using the catalyst. This object is achieved by a material composition for producing a dehydrogenation catalyst according to claim 1, a process for producing a calcined dehydrogenation catalyst according to claim 6, and a method according to claim 7.

MEANS FOR SOLVING THE PROBLEM

The inventors have diligently investigated and found that, among cerium compounds, cerium carbonate hydroxide is suitable as a cerium compound, which is easy to handle in case that it is used in preparation of a catalyst wherein only a small amount of moisture is contained and its particle diameter is in the order of a few $\mu$m, also which has a high performance when it is used in a dehydrogenation catalyst as a cerium source.

By using cerium carbonate hydroxide as a cerium source, they have succeeded in preparing a high cerium-containing dehydrogenation catalyst containing 5 - 35 % by weight of $CeO_2$, wherein sufficient physical strength and catalytic performance are achieved. Moreover, the cost can be kept at the same level because cerium carbonate hydroxide can be handled like cerium compounds conventionally used in the production of a dehydrogenation catalyst.

ADVANTAGEOUS EFFECT OF THE INVENTION

The present invention enables the production of dehydrogenation catalyst pellets having sufficient physical strength durable for the industrial use by using only cerium carbonate hydroxide as a cerium source, or cerium carbonate hydroxide mixed with other cerium compounds, in a high cerium-containing dehydrogenation catalyst comprising iron oxide and potassium oxide.

BEST MODE FOR CARRYING OUT THE INVENTION

The present invention is described below in detail.

The cerium carbonate hydroxide used in the present invention are characterized such that the amount contained in the oxide is greater or equal to 60 %, more preferably greater or equal to 65 %, and its particle diameter is 0.1 - 30

$\mu$m, more preferably 0.5 - 5 $\mu$m.

The cerium carbonate hydroxide (Cerium Carbonate Hydroxide, $CeCO_3OH$ or Cerium Carbonate Hydroxide Hydrate, $Ce_2(CO_3)_2(OH)_2 \cdot H_2O$) used in the present invention is also called Basic Cerium Carbonate or Cerium Hydroxycarbonate. Also, it is sometimes called Cerium Oxide Carbonate Hydrate and described as $Ce(CO_3)_2O \cdot H_2O$ or $Ce_2O(CO_3)_2 \cdot H_2O$ or $CeO(CO_3)_2 \cdot x\ H_2O)$ and the like. If the characteristics are similar to the above, any names and chemical formulae can be used.

Catalytic components converted into its oxide in percentage on the basis of total weight of the catalyst are in the range as follows:

$Fe_2O_3$ 35.0 - 85.0 % by weight

$K_2O$ 5.0 - 30.0 % by weight

$CeO_2$ 5.0 - 35.0 % by weight

Furthermore, as a promoter, it contains 0.0001 - 6.0 % by weight of at least one oxide selected from the group consisting of magnesium, calcium, titanium, zirconium, vanadium, niobium, chrome, molybdenum, tungsten, manganese, rhenium, ruthenium, osmium, cobalt, rhodium, iridium, nickel, palladium, platinum, copper, silver, gold, zinc, boron, aluminum, gallium, indium, silicon, germanium, stannum, phosphorus, antimony, bismuth, lanthanum, praseodymium, neodymium and samarium, respectively on the basis of total weight of the catalyst.

The iron oxides used in the present invention can be red, yellow, brown or black iron oxides in different conformation, especially, red iron oxide ($Fe_2O_3$) is preferable. A mixture of some iron oxides such as a mixture of yellow iron oxide ($Fe_2O_3 \cdot H_2O$) and red iron oxide can be used as well.

As to the potassium compounds used in the present invention, oxides, hydroxides, carbonates, bicarbonates and the like and any mixture thereof are preferable, especially, potassium carbonate or a mixture of potassium carbonate and potassium oxide is the most preferable.

As to the cerium compounds used in the present invention, cerium carbonate hydroxide or a mixture of cerium carbonate hydroxide and other cerium compounds are used. Other cerium compounds include cerium oxide, cerium hydroxide, cerium carbonate, cerium nitrate and any mixture thereof.

Components to be added as promoters are not necessarily limited to oxides. Any components which are thermally decomposable can be used, however, it is required that the components do not comprise any catalyst poisons such as sulfur and the like.

Catalyst materials including iron dioxide are subject to wet kneading. In this process, it is possible to use only cerium carbonate hydroxide as a source of cerium, but also a mixture of cerium carbonate hydroxide and other cerium compounds can be used. In the latter case, the physical strength of dehydrogenation catalyst pellets to be obtained in further process is sometimes improved.

The amount of moisture to be added in kneading process should be the amount suitable to the following extrusion molding process. The amount differs depending on the material to be used. Generally, in the range of 2 - 50 % by weight of water is added and subjected to sufficient kneading, and then subjected to extrusion molding then dried and calcined, as a result, the desired dehydrogenation catalyst pellets are obtained. Drying of the mixture is carried out only to the extent to remove free water contained in the extrusion molded product, generally carried out at 70 - 200 °C, more preferably at 100 - 150 °C. On the other hand, calcination is carried out to decompose each catalyst precursor contained in the dried product and to improve the physical stability of the catalyst pellets as well as to improve its performance, generally carried out at in the range of 400 - 1000 °C, more preferably at in the range of 500 - 900 °C.

The dehydrogenation catalyst for dehydrogenating alkyl aromatic compounds of the present invention is effective as a dehydrogenation catalyst producing vinyl aromatic compounds by contacting alkyl aromatic compound with water vapor. It is effective especially in promoting dehydrogenation of ethylbenzene in producing styrene by contacting ethylbenzene with water vapor and it stabilizes the dehydrogenation reaction in the presence of steam physically.

The pellets comprising the above-mentioned dehydrogenation catalyst composition of the present invention has catalyst performance equal to the conventional dehydrogenation catalytic pellets, and its crash strength in resting state and its abrasion strength in moving state are doubled respectively. Namely, considerably high physical strength wherein the crash strength is 20 - 50 N/mm and the abrasion strength is 0.3 - 4 % can be achieved and a catalyst which is durable for an industrial use can be obtained.

The following examples describe the present invention in more detail but the invention is not limited to those examples.

EXAMPLE 1

All materials of a dehydrogenation catalyst for dehydrogenating alkyl aromatic compounds used were commercially available products and a catalyst was prepared as follows: 500 g of red iron oxide, 106 g of potassium carbonate, 21 g of calcium hydroxide, 19 g of molybdenum oxide and 217 g of cerium carbonate hydroxide were weighed and introduced into a kneader, mixed and processed to a paste by gradually adding pure water, then the resulting product was extruded and molded into cylindrical pellets having 3 mm diameter, dried for several hours in a dryer, then transferred into an electrical furnace and calcined for 2 hours at 900 °C.

The obtained catalyst had the following compositions:

| | |
|---|---|
| $Fe_2O_3$ | 66.0 % by weight |
| $K_2O$ | 9.5 % by weight |
| $CeO_2$ | 20.0 % by weight |
| CaO | 2.0 % by weight |
| $MoO_3$ | 2.5 % by weight |

EXAMPLE 2

A catalyst was prepared according to the procedure of Example 1, except that the amount of cerium carbonate hydroxide was changed to 108 g and 152 g of cerium carbonate were added in the wet kneading process of catalyst materials including iron oxide. The compositions of the catalyst were also the same as Example 1.

COMPARATIVE EXAMPLE 1

A catalyst was prepared according to the procedure of Example 1, except that cerium carbonate hydroxide was not added but 303 g of cerium carbonate were added as a cerium source in the wet kneading process of catalyst materials including iron oxide. The compositions of the catalyst were also the same as Example 1.

EXPERIMENTAL EXAMPLE

Crash strength measurement and abrasion strength measurement were carried out to measure the physical strength.

Crash strength is a property indicating a compression strength of catalytic pellets, wherein a catalytic particle is contacted at the ridge line and it is subject to weight bearing gradually from above and the force required to destroy the catalyst pellets (N) is measured, crash strength (N/mm) is obtained by dividing N by length of a catalyst (mm) and expressed in an average value of 25 catalyst pellets. As a measurement apparatus, a Chatillon hardness tester, model TCD500 made by Chatillon was used.

Abrasion strength indicates abrasion resistance and it indicates the strength in moving state while crash strength indicates the strength in resting state. Catalytic pellets were sieved with a 20 mesh standard sieve and then 40 g thereof was weighed and taken. The pellets were introduced into a cylindrical LOA measurement container having 275 mm of inner diameter and 260 mm of length equipped with one baffle with a height of 25.4 mm and a length of 260 mm and then rotated for 30 minutes at 56 rpm. The pellets were sieved with a 20 mesh standard sieve and then the weight of the remaining pellets on the sieve was measured, abrasion strength (%) was calculated by the following formula:

$$\text{Abrasion strength (\%)} = (40g - \text{the weight of the remaining pellets on the sieve (g))} / 40g \times 100$$

Performance evaluation was carried out under the following conditions.

| | |
|---|---|
| $H_2O$/ethylbenzene (ratio by weight) | 2.0 |
| Reaction temperature (°C) | 620, 600, 570, 540 |

As for performance evaluation, a conversion rate was derived from the ethylbenzene concentration (wt%) at entry and exit points of catalyst layer and a selectivity rate was derived from the ethylbenzene concentration (wt%) at entry and exit points of catalyst layer and styrene concentration (wt%) at exit point of catalyst layer. Performance evaluation was expressed in T60 (reaction temperature indicating 60 % of conversion rate) and S60 (selectivity at 60 % of conversion rate).

The physical strength and the performance evaluation of each dehydrogenation catalyst are given in Table 1 below.

[Table 1]

| Physical Strength and Catalyst Performance of Each Dehydrogenation Catalyst | | | | |
|---|---|---|---|---|
| | Crash Strength (N/mm) | Abrasion Strength (%) | T60 (°C) | S60 (wt%) |
| Example 1 | 45 | 3.7 | 576 | 97.3 |
| Example 2 | 36 | 1.5 | 573 | 97.2 |
| Comparative Example 1 | 17 | 8.0 | 578 | 97.3 |

The result of Table 1 demonstrates that the physical strength of the pellets of dehydrogenation catalyst using cerium carbonate hydroxide as a cerium source in the alkyl aromatic dehydrogenating catalyst or those using a mixture of cerium carbonate hydroxide and cerium carbonate was improved to obtain the strength almost twice as much as the pellets of dehydrogenation catalyst using cerium carbonate. It was confirmed that they were not only sufficiently adoptable for industrial use but also they were almost at the same level in performance.

## Claims

1. A dehydrogenation catalyst for dehydrogenating alkyl aromatic compounds of which the final catalyst composition comprises 35.0 - 85.0 % by weight of iron oxide calculated as $Fe_2O_3$, 5.0 - 30.0 % by weight of potassium compounds calculated as $K_2O$, and 5.0 - 35.0 % by weight of cerium compounds calculated as $CeO_2$, and which is obtained by using only cerium carbonate hydroxide as a cerium source or cerium carbonate hydroxide mixed with other cerium compounds.

2. The dehydrogenation catalyst for dehydrogenating alkyl aromatic compounds of Claim 1 wherein the iron oxide is red iron oxide ($Fe_2O_3$) or a mixture of red iron oxide and yellow iron oxide ($Fe_2O_3 \cdot H_2O$).

3. The dehydrogenation catalyst for dehydrogenating alkyl aromatic compounds of Claim 1 wherein the potassium compounds are oxides, hydroxides, carbonates, bicarbonates and any combinations thereof.

4. The dehydrogenation catalyst for dehydrogenating alkyl aromatic compounds of Claim 1 wherein the other cerium compounds are cerium oxide, cerium hydroxide, cerium carbonate, cerium nitrate, and any combinations thereof.

5. The dehydrogenation catalyst for dehydrogenating alkyl aromatic compounds of Claim 1 further comprising respectively 0.0001 - 6.0 % by weight of at least one oxide or compound as a promoter selected from the group consisting of magnesium, calcium, titanium, zirconium, vanadium, niobium, chrome, molybdenum, tungsten, manganese, rhenium, ruthenium, osmium, cobalt, rhodium, iridium, nickel, palladium, platinum, copper, silver, gold, zinc, boron, aluminum, gallium, indium, silicon, germanium, stannum, phosphorus, antimony, bismuth, lanthanum, praseodymium, neodymium and samarium.

6. A process for producing a calcined dehydrogenation catalyst for dehydrogenating alkyl aromatic compounds comprising the following steps of;
   preparing an extrudable mixture by admixing compositions of a dehydrogenation catalyst for dehydrogenating alkyl aromatic compounds of Claim 1 with sufficient water to form an extrudable mixture;
   molding the extrudable mixture into pellets; and
   drying and calcining the pellets to be a finished catalyst.

7. A method for dehydrogenation of alkyl aromatic compounds to produce vinyl aromatic compounds by contacting alkyl aromatic compounds with water vapor in the presence of the dehydrogenation catalyst for dehydrogenating alkyl aromatic compounds obtained by the process of Claim 6.

8. The method for dehydrogenation of Claim 7, wherein the alkyl aromatic compound is ethylbenzene and the vinyl aromatic compound is styrene.

**Patentansprüche**

1. Dehydrierungskatalysator zur Dehydrierung alkylaromatischer Verbindungen, wobei die Endzusammensetzung des Katalysators 35,0 - 85,0 Gewichts-% als $Fe_2O_3$ berechnetes Eisenoxid, 5,0 - 30,0 Gewichts-% als $K_2O$ berechnete Kaliumverbindungen und 5,0 - 35,5 Gewichts-% als $CeO_2$ berechnete Cerverbindungen umfasst, und der nur unter Verwendung von Cercarbonat - hydroxid als Cerquelle oder mit anderen Cerverbindungen gemischtes Cercarbonat -hydroxid erhalten wird.

2. Dehydrierungskatalysator zur Dehydrierung alkylaromatischer Verbindungen nach Anspruch 1, wobei das Eisenoxid rotes Eisenoxid ($Fe_2O_3$) oder eine Mischung aus rotem Eisenoxid und gelbem Eisenoxid ($Fe_2O_3 \cdot H_2O$) ist.

3. Dehydrierungskatalysator zur Dehydrierung alkylaromatischer Verbindungen nach Anspruch 1, wobei die Kaliumverbindungen Oxide, Hydroxide, Carbonate, Bicarbonate und beliebige Kombinationen davon sind.

4. Dehydrierungskatalysator zur Dehydrierung alkylaromatischer Verbindungen nach Anspruch 1, wobei die anderen Cerverbindungen Ceroxid, Cerhydroxid, Cercarbonat, Cernitrat und beliebige Kombinationen davon sind.

5. Dehydrierungskatalysator zur Dehydrierung alkylaromatischer Verbindungen nach Anspruch 1, weiterhin umfassend jeweils 0,0001 -6,0 Gewichts-% wenigstens eines Oxids oder einer Verbindung als Promotor, ausgewählt aus der Gruppe bestehend aus Magnesium, Calcium, Titan, Zirkon, Vanadium, Niob, Chrom, Molybdän, Wolfram, Mangan, Rhenium, Ruthenium, Osmium, Cobalt, Rhodium, Iridium, Nickel, Palladium, Platin, Kupfer, Silber, Gold, Zink, Bor, Aluminium, Gallium, Indium, Silizium, Germanium, Zinn, Phosphor, Antimon, Bismut, Lanthan, Praseodym, Neodym und Samarium.

6. Verfahren zur Herstellung eines kalzinierten Dehydrierungskatalysators zur Dehydrierung alkylaromatischer Verbindungen, umfassend die folgenden Schritte des
   Herstellens einer extrudierbaren Mischung durch Vermischen von Zusammensetzungen eines Dehydrierungskatalysators zur Dehydrierung alkylaromatischer Verbindungen nach Anspruch 1 mit ausreichend Wasser unter Bildung einer extrudierbaren Mischung,
   Formens der extrudierbaren Mischung zu Pellets und des Trocknens und Kalzinierens der Pellets zum fertigen Katalysator.

7. Verfahren zur Dehydrierung alkylaromatischer Verbindungen zur Herstellung vinylaromatischer Verbindungen, indem alkylaromatische Verbindungen mit Wasserdampf in Gegenwart des Dehydrierungskatalysators zur Dehydrierung alkylaromatischer Verbindungen, die mit dem Verfahren nach Anspruch 6 erhalten werden, in Kontakt gebracht werden.

8. Verfahren zur Dehydrierung nach Anspruch 7, wobei die alkylaromatische Verbindung Ethylbenzol und die vinylaromatische Verbindung Styrol ist.

**Revendications**

1. Catalyseur de déshydrogénation pour la déshydrogénation de composés aromatiques alkyliques dont la composition de catalyseur finale comprend 35,0 à 85,0 % en poids d'oxyde de fer calculé en tant que $Fe_2O_3$, 5,0 à 30,0 % en poids de composés de potassium calculés en tant que $K_2O$ et 5,0 à 35,0 % en poids de composés de cérium calculés en tant que $CeO_2$, et qui est obtenu en utilisant uniquement de l'hydroxyde de carbonate de cérium en tant que source de cérium ou de l'hydroxyde de carbonate de cérium mélangé à d'autres composés de cérium.

2. Catalyseur de déshydrogénation pour la déshydrogénation de composés aromatiques alkyliques selon la revendication 1, dans lequel l'oxyde de fer est l'oxyde de fer rouge ($Fe_2O_3$) ou un mélange d'oxyde de fer rouge et d'oxyde de fer jaune ($Fe_2O_3 \cdot H_2O$).

3. Catalyseur de déshydrogénation pour la déshydrogénation de composés aromatiques alkyliques selon la revendi-

EP 2 106 852 B1

cation 1, dans lequel les composés de potassium sont des oxydes, hydroxydes, carbonates, bicarbonates et toute combinaison de ceux-ci.

4. Catalyseur de déshydrogénation pour la déshydrogénation de composés aromatiques alkyliques selon la revendication 1, dans lequel les autres composés de cérium sont l'oxyde de cérium, l'hydroxyde de cérium, le carbonate de cérium, le nitrate de cérium et toute combinaison de ceux-ci.

5. Catalyseur de déshydrogénation pour la déshydrogénation de composés aromatiques alkyliques selon la revendication 1, comprenant en outre 0,0001 à 6,0 % en poids respectivement d'au moins un oxyde ou composé comme promoteur sélectionné parmi le groupe constitué du magnésium, calcium, titane, zirconium, vanadium, niobium, chrome, molybdène, tungstène, manganèse, rhénium, ruthénium, osmium, cobalt, rhodium, iridium, nickel, palladium, platine, cuivre, argent, or, zinc, bore, aluminium, gallium, indium, silicium, germanium, étain, phosphore, antimoine, bismuth, lanthane, praséodyme, néodyme et samarium.

6. Procédé de production d'un catalyseur de déshydrogénation calciné pour la déshydrogénation de composés aromatiques alkyliques comprenant les étapes suivantes consistant à :

préparer un mélange pouvant être extrudé en mélangeant des compositions d'un catalyseur de déshydrogénation pour la déshydrogénation de composés aromatiques alkyliques selon la revendication 1 à suffisamment d'eau pour former un mélange pouvant être extrudé ;
mouler le mélange pouvant être extrudé en des pellets ; et
sécher et calciner les pellets pour être un catalyseur fini.

7. Procédé de déshydrogénation de composés aromatiques alkyliques pour produire des composés aromatiques vinyliques en mettant en contact des composés aromatiques alkyliques avec de la vapeur d'eau en présence du catalyseur de déshydrogénation pour la déshydrogénation de composés aromatiques alkyliques obtenu par le procédé selon la revendication 6.

8. Procédé de déshydrogénation selon la revendication 7, dans lequel le composé aromatique alkylique est l'éthylbenzène et le composé aromatique vinylique est le styrène.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0297657 A1
- JP 3011812 A
- US 4758543 A
- US 5376613 A
- JP 5105428 A
- JP 2000159521 A
- JP 2003238948 A